Europäisches Patentamt

European Patent Office

Office européen des brevets

(11) Veröffentlichungsnummer: **0 535 489 B1**

(12) **EUROPÄISCHE PATENTSCHRIFT**

(45) Veröffentlichungstag der Patentschrift: **21.06.95**

(51) Int. Cl.⁶: **C07C 57/04**, C07C 51/25

(21) Anmeldenummer: **92116095.8**

(22) Anmeldetag: **21.09.92**

(54) **Verfahren zur katalytischen Gasphasenoxidation von Methacrolein zu Methacrylsäure.**

(30) Priorität: **01.10.91 DE 4132684**

(43) Veröffentlichungstag der Anmeldung:
**07.04.93 Patentblatt 93/14**

(45) Bekanntmachung des Hinweises auf die
Patenterteilung:
**21.06.95 Patentblatt 95/25**

(84) Benannte Vertragsstaaten:
**BE DE ES FR GB IT**

(56) Entgegenhaltungen:
**EP-A- 0 467 144**
**EP-A- 0 534 294**
**DE-A- 2 238 851**
**DE-A- 2 513 405**

**PATENT ABSTRACTS OF JAPAN no. 890 (C-78)(20) 1978 & JP-A-53 031 615 (NIPPON KAYAKU K.K.) 25. März 1978**

**PATENT ABSTRACTS OF JAPAN vol. 13, no. 419 (C-637)(3767) 18. September 1989 & JP-A-01 157 930 (MITSUI TOATSU CHEM. INC.) 21. Juni 1989**

(73) Patentinhaber: **BASF Aktiengesellschaft**
**Carl-Bosch-Strasse 38**
**D-67063 Ludwigshafen (DE)**

(72) Erfinder: **Hammon, Ulrich, Dr.**
**Nietzschestrasse 30**
**W-6800 Mannheim 1 (DE)**
Erfinder: **Herzog, Klaus, Dr.**
**Gronauer Strasse 49**
**W-6700 Ludwigshafen (DE)**
Erfinder: **Neumann, Hans-Peter, Dr.**
**Der Weidenweg 29**
**W-6800 Mannheim 31 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur katalytischen Gasphasenoxidation von Methacrolein zu Methacrylsäure in einem Kontaktrohr-Festbettreaktor bei erhöhter Temperatur an katalytisch aktiven Oxiden mit einem Umsatz des Methacroleins bei einfachem Durchgang von 40 bis 95 mol%.

Sowohl Methacrylsäure für sich, als auch mit niederen Alkoholen verestert, eignet sich als Ausgangsmonomeres zur Herstellung von Polymerisaten verschiedenster Anwendungszwecke (z.B. Klebstoffe).

Die Herstellung von Methacrylsäure durch katalytische Gasphasenoxidation von Methacrolein verläuft stark exotherm, weshalb es infolge einer Vielfalt von möglichen Parallel- oder Folgereaktionen im Hinblick auf eine möglichst selektive Umsetzung des Methacroleins zu Methacrylsäure notwendig ist, den Verlauf der Reaktionstemperatur zu steuern.

Aus der DE-A 22 51 364 ist bekannt, bei Methacroleinumsätzen bei einfachem Durchgang von unter 10 bis über 95 % den Verlauf der Reaktionstemperatur der katalytischen Gasphasenoxidation von Methacrolein zu Methacrylsäure in einem Kontaktroh-Festbettreaktor so zu steuern, daß man die Kontaktrohre mit einer 330 oder 340 °C aufweisenden Salzschmelze umgibt. Nachteilig an diesem Verfahren ist jedoch, daß die so implizit längs der Kontaktrohre eingestellten Verläufe der Reaktionstemperatur im Hinblick auf eine möglichst selektive Umsetzung des Methacroleins zur Methacrylsäure, insbesondere im Umsatzbereich von 40 bis 95 % an umgesetztem Methacrolein (bei einfachem Durchgang), nicht voll zu befriedigen vermögen.

Aufgabe der vorliegenden Erfindung war daher, ein Verfahren zur katalytischen Gasphasenoxidation von Methacrolein zu Methacrylsäure in einem Kontaktrohr-Festbettreaktor bei erhöhter Temperatur an katalytisch aktiven Oxiden mit einem Umsatz des Methacroleins bei einfachem Durchgang von 40 bis 95 % zur Verfügung zu stellen, das einen im Hinblick auf eine erhöhte Selektivität der Methacrylsäurebildung verbesserten Verlauf der Reaktionstemperatur aufweist.

Demgemäß wurde ein Verfahren zur katalytischen Gasphasenoxidation von Methacrolein zu Methacrylsäure in einem Kontaktrohr-Festbettreaktor bei erhöhter Temperatur an katalytisch aktiven Oxiden mit einem Umsatz des Methacroleins bei einfachem Durchgang von 45 bis 95 % gefunden, das dadurch gekennzeichnet ist, daß die Reaktionstemperatur in Strömungsrichtung längs der Kontaktrohre (längs der Reaktionsachse) in einer ersten Reaktionszone ab Eintritt der die Reaktanden enthaltenden Ausgangsreaktionsgase in die Kontaktrohre bis zum Erreichen eines Umsatzes des Methacroleins von 20 bis 40 % 280 bis 340 °C beträgt und anschließend die Reaktionstemperatur abrupt oder längs der Kontaktrohre bis zum Erreichen eines Methacroleinumsatzes von 45 bis 95 %, d.h. bis zum Austritt der Reaktionsgase aus den Kontaktrohren, sukzessive stufenweise oder kontinuierlich um insgesamt 5 bis 45 °C mit der Maßgabe gesenkt wird, daß die Reaktionstemperatur in dieser zweiten Reaktionszone nicht weniger als 260 °C beträgt.

Als oxidische Katalysatoren eignen sich unter anderen die Massen, die in der EP-A 265 733, der EP-A 102 688 und der DE-A 30 10 434 beschrieben sind.

Vorzugsweise werden Massen der allgemeinen Formel I

$$Mo_{12}P_aX^1_bX^2_cX^3_dX^4_eX^5_fO_n \qquad (I),$$

in der die Variablen folgende Bedeutung haben:

X$^1$: wenigstens ein Alkalimetall und/oder Erdalkalimetall,
X$^2$: Arsen, Wolfram, Niob und/oder Vanadin,
X$^3$: Antimon, Wismut, Zirkonium und/oder Bor,
X$^4$: Chrom, Cobalt, Mangan, Zinn, Schwefel, Zink, Silicium, Selen, Eisen und/oder Nickel,
X$^5$: Kupfer, Silber, Rhodium und/oder Rhenium,
a: 0,3 bis 3
b: 0 bis 3
c: 0 bis 3
d: 0 bis 3
e: 0 bis 3
f: 0 bis 2 und
n: eine Zahl, die durch die Wertigkeit und Häufigkeit der von Sauerstoff verschiedenen Elementen in I bestimmt wird,

eingesetzt.

Mit besonderem Vorteil eignen sich Massen der allgemeinen Formel II,

$$Mo_{12}P_aV_bX^1_cX^2_dX^3_eSb_fRe_gS_hO_n \qquad (II),$$

in der die Varianten folgende Bedeutung haben:

$X^1$: Kalium, Rubidium und/oder Cäsium,

$X^2$: Kupfer und/oder Silber,

$X^3$: Cer, Bor, Zirkonium, Mangan und/oder Wismut,

a: 0,5 bis 3,0

b: 0,01 bis 3,0

c: 0,2 bis 3,0

d: 0,01 bis 2,0

e: 0 bis 2,0

f: 0,01 bis 2,0

g: 0 bis 1,0

h: 0,001 bis 0,5 und

n: eine Zahl, die durch die Wertigkeit und Häufigkeit der von Sauerstoff verschiedenen Elemente in II bestimmt wird,

wobei unter den Massen der allgemeinen Formel II diejenige der Zusammensetzung

$$Mo_{12}P_2V_1Cs_2K_{0,03}Cu_{0,5}Sb_1S_{0,03}Re_{0,04}$$

besonders bevorzugt ist.

Die genannten oxidischen Katalysatoren sind in an sich bekannter Weise erhältlich. Sie können beispielsweise dadurch hergestellt werden, daß man als Ausgangsverbindungen geeignete Salze der sie konstituierenden elementaren Bestandteile, gegebenenfalls bei erhöhter Temperatur und unter Zusatz von Säuren oder Basen, in wäßrigem Medium durch Lösen und/oder Suspendieren fein verteilt, mischt, das Gemisch trocknet, die erhaltene Masse formt und in der Regel bei Temperaturen von 180 bis 480, vorzugsweise 350 bis 450° C im Luftstrom oder in inerter Atmosphäre, z.B. $N_2$ oder $CO_2$, calciniert. Bei der Formung können an sich bekannte Hilfsmittel wie Gleitmittel (z.B. Graphit) oder Formhilfsmittel und Verstärkungsmittel wie Mikrofasern aus Glas, Asbest, Siliciumcarbid oder Kaliumtitanat zugesetzt werden. In dieser Form werden die oxidischen Massen zweckmäßigerweise zur Verwendung als Vollkatalysatoren hergestellt, wobei Hohlzylinder mit einem Außendurchmesser und einer Länge von 4 bis 10 mm und einer Wandstärke von 1 bis 3 mm die bevorzugte Katalysatorgeometrie darstellen. Nähere Ausführungen findet man in der älteren Anmeldung DE-P 4 022 212.8. Die katalytisch aktiven Oxide können aber auch in Gestalt von Schalenkatalysatoren, das heißt auf ein vorgeformtes Trägermaterial aufgebracht, angewendet werden, wobei das Aufbringen auf das Trägermaterial, z.B. in Form einer wäßrigen Ausgangslösung oder Suspension, verbunden mit anschließender Trocknung und Calcinierung, oder als bereits calcinierte pulverisierte Masse in Kombination mit einem Bindemittel erfolgen kann.

Selbstverständlich können die katalytisch aktiven oxidischen Massen auch in Pulverform als Katalysatoren eingesetzt werden.

Der zur Oxidation des Methacroleins benötigte Sauerstoff kann z.B. in Form von Luft, aber auch in reiner Form eingesetzt werden. Aufgrund der hohen Reaktionswärme werden die Reaktionspartner vorzugsweise mit Inertgas wie $N_2$, rückgeführten Reaktionsabgasen und/oder Wasserdampf verdünnt. In der Regel wird bei einem Methacrolein : Sauerstoff : Wasserdampf : Inertgas Volumen (Nl)-Verhältnis von 1:(1 bis 3):(2 bis 20):(3 bis 30), vorzugsweise von 1:(1 bis 3):(3 bis 10):(7 bis 18) gearbeitet. Der Reaktionsdruck liegt üblicherweise im Bereich von 1 bis 3 bar und die Gesamtraumbelastung beträgt vorzugsweise 800 bis 1800 Nl/l/h. Mit Vorteil wird bei dem erfindungsgemäßen Verfahren Methacrolein eingesetzt, das in an sich bekannter Weise durch Kondensation von Propanol mit Formaldehyd in Gegenwart von sekundären Aminen und Säuren in flüssiger Phase erhältlich ist. Bei dem beschriebenen Verfahren wird keine reine Methacrylsäure erhalten, sondern ein Gasgemisch, von dessen Nebenkomponenten die Methacrylsäure in an sich bekannter Weise abgetrennt werden kann.

Die Realisierung des erfindungsgemäßen Profils der Reaktionstemperatur kann in an sich bekannter Weise erfolgen, z.B. durch abschnittsweise Heizung oder Kühlung des Kontaktrohres mittels elektrischer Temperierbänder oder zirkulierenden fluiden Temperaturmedien wie Schmelzen von Salzen wie Kaliumnitrat, Natriumnitrit und/oder Natriumnitrat, oder von niedrig schmelzenden Metallen wie Natrium, Zinn, Quecksilber sowie Legierungen verschiedener Metalle oder Wärmeträgerölen, wobei im Falle eines Einzelrohres infolge des hohen Wärmeübergangs die während der Reaktion im Rohrinneren herrschende Temperatur im wesentlichen gleich der äußeren Heiz- oder Kühltemperatur ist.

Abschnittsweises Heizen oder Kühlen ist aber auch bei Vielrohr-Festbettreaktoren möglich, wie sie zur großtechnischen Realisierung des erfindungsgemäßen Verfahrens vorzugsweise eingesetzt werden, und z.B.

in den Schriften DE-A 28 30 765, DE-A 22 01 528, DE-A 16 01 162, DE-A 25 13 405 sowie US-A 3 147 084 beschrieben sind. Eine weitere Möglichkeit der Steuerung der Reaktionstemperatur besteht im abschnittsweisen Erhöhen oder Senken der Katalysatoraktivität. Dies kann durch chemische Modifikation der aktiven Katalysatormasse, aber auch durch Verdünnen mit desaktiviertem Katalysator oder Inertmaterial realisiert werden. Gegebenenfalls kann auch abschnittsweises Heizen/Kühlen mit abschnittsweisem Erhöhen/Senken der Katalysatoraktivität kombiniert werden. Besonders günstige Selektivitäten der Methacrylsäurebildung werden dann erhalten, wenn bei einem Methacroleinumsatz von 50 bis 75 % gearbeitet wird. Mit besonderem Vorteil wird so gearbeitet, daß im Anschluß an die erste Reaktionszone die Reaktionstemperatur abrupt oder längs der Reaktionsachse bis zum Erreichen des Methacroleinendumsatzes sukzessive stufenweise oder kontinuierlich um insgesamt 10 bis 40 °C gesenkt wird. Anwendungstechnisch bevorzugt ist eine stufenweise Absenkung, wobei in der Regel 2 bis 4 Stufen angewendet werden.

Da in der Praxis, insbesondere im Falle des mittels Salzbad temperierten Vielrohr-Festbettreaktors, nur ein endlicher Wärmeübergang vom Temperaturmedium auf die Reaktionsgase erreicht werden kann, hat es sich als vorteilhaft erwiesen, die Ausgangreaktionsgase der ersten Reaktionszone vorgeheizt, in der Regel auf Reaktionstemperatur, zuzuführen. Wird längs der ersten Reaktionszone eine Temperatur des Temperiermediums oberhalb 280 °C, aber ≤310 °C gewählt, werden die Reaktionsgase der ersten Reaktionszone vorzugsweise auf eine Temperatur vorgeheizt zugeführt, die 20 bis 30 °C oberhalb der Temperatur liegt, die das Temperiermedium am Beginn der ersten Reaktionszone aufweist. Liegt die Temperatur des Temperiermediums längs der ersten Reaktionszone im Bereich >310 °C aber ≤340 °C, werden die Reaktionsgase der ersten Reaktionszone vorzugsweise auf eine Temperatur vorgeheizt zugeführt, die lediglich bis zu 15 °C oberhalb der Temperatur liegt, die das Temperiermedium am Beginn der ersten Reaktionszone aufweist. Ist die Temperatur des Temperiermediums längs der ersten Reaktionszone konstant, stellt sich so in Strömungsrichtung längs der ersten Reaktionszone eine kontinuierlich fallende Reaktionstemperatur ein. Dies hat sich als besonders vorteilhaft erwiesen. Insbesondere dann, wenn der kontinuierliche Abfall der Reaktionstemperatur längs der zweiten Reaktionszone in Strömungsrichtung fortgesetzt wird. Selbstverständlich kann der kontinuierliche Abfall der Reaktionstemperatur längs der Reaktionsachse durch sukzessive stufenweise Temperaturabnahme angenähert werden. Vorzugsweise beträgt der Abfall der Reaktionstemperatur längs der ersten Reaktionszone in Strömungsrichtung 5 bis 40, besonders bevorzugt 15 bis 40 °C.

Typische Kontaktrohre bestehen aus korrosions- und hitzebeständigem Stahl (z.B. V2A) einer Wanddikke von etwa 2 mm und einem Innendurchmesser von 25 mm. Die Anzahl solcher Kontaktrohre in einem Vielrohr-Festbettreaktor beläuft sich in der Regel auf 10 000 bis 40 000. Umsatz U und Selektivität S sind in dieser Schrift wie folgt definiert:

$$U = \frac{\text{Molzahl umgesetztes Methacrolein}}{\text{Molzahl eingesetztes Methacrolein}} \times 100$$

$$S = \frac{\text{Molzahl gebildete Methacrylsäure}}{\text{Molzahl umgesetztes Methacrolein}} \times 100 \ .$$

Beispiele B1 bis B4 und Vergleichsbeispiele V1 bis V2

Ein mittels elektrischer Heizbänder abschnittsweise temperierbares Stahlrohr (V2A, 2 mm Wanddicke, 25 mm Innendurchmesser) wurde mit dem Vollkatalysator entsprechend Beispiel 1 der älteren Anmeldung DE-P 4 022 212.8 bis zu einer Füllhöhe von 3 m gefüllt und mit 1200 Nl/l/h einer Gasmischung der Zusammensetzung

4,5 Vol.% Methacrolein

9,5 Vol.% Sauerstoff

24 Vol.% Wasserdampf und

62 Vol.% Stickstoff,

die je nach Beispiel auf verschiedene Temperaturen $T^1$ vorgeheizt war, beschickt. Anschließend wurde längs der ersten Reaktionszone bis zu einem Methacroleinumsatz von 40 % die Temperatur der elektri-

schen Heizbänder auf $T^2$ und danach bis zum Verlassen der Reaktionsgase auf $T^3$ eingestellt. Der Endumsatz $U^{End}$ wurde durch die Länge der zweiten Reaktionszone determiniert. Die erhaltenen Ergebnisse (Selektivität $S^{End}$ der Methacrylsäurebildung) weist die Tabelle aus.

|  | $T^1$ | $T^2$ | $T^3$ | $U^{End}$ | $S^{End}$ |
|---|---|---|---|---|---|
| B1 | 300 | 300 | 286 | 69 | 87,3 |
| B2 | 310 | 310 | 281 | 70 | 88,5 |
| B3 | 320 | 320 | 278 | 69 | 87,0 |
| B4 | 330 | 310 | 279 | 70 | 88,7 |
| V1 | 290 | 290 | 290 | 68 | 85,4 |
| V2 | 270 | 270 | 328 | 69 | 81,3 |

**Patentansprüche**

1. Verfahren zur katalytischen Gasphasenoxidation von Methacrolein zu Methacrylsäure in einem Kontaktrohr-Festbettreaktor bei erhöhter Temperatur an katalytisch aktiven Oxiden mit einem Umsatz des Methacroleins bei einfachem Durchgang von 40 bis 95 mol%, dadurch gekennzeichnet, daß die Reaktionstemperatur in Strömungsrichtung längs der Kontaktrohre in einer ersten Reaktionszone ab Eintritt der die Reaktanden enthaltenden Ausgangsreaktionsgase in die Kontaktrohre bis zum Erreichen eines Umsatzes des Methacroleins von 20 bis 40 % 280 bis 340°C beträgt und anschließend die Reaktionstemperatur abrupt oder längs der Kontaktrohre sukzessive stufenweise oder kontinuierlich bis zum Erreichen eines Methacroleinumsatzes von 45 bis 95 % um insgesamt 5 bis 45°C mit der Maßgabe gesenkt wird, daß die Reaktionstemperatur in dieser zweiten Reaktionszone nicht weniger als 260°C beträgt.

2. Verfahren nach Anspruch 1, dadurch gekennzeichnet, daß die Reaktionstemperatur der Reaktionsgase in Strömungsrichtung längs der Kontaktrohre bis zum Verlassen der Kontaktrohre sukzessive abnimmt.

3. Verfahren nach Anspruch 1 oder 2, dadurch gekennzeichnet, daß als oxidische Katalysatoren Massen der allgemeinen Formel I

$$Mo_{12}P_aX^1_bX^2_cX^3_dX^4_eX^5_fO_n \qquad (I),$$

in der die Variablen folgende Bedeutung haben:

$X^1$: wenigstens ein Alkalimetall und/oder Erdalkalimetall,
$X^2$: Arsen, Wolfram, Niob und/oder Vanadin,
$X^3$: Antimon, Wismut, Zirkonium und/oder Bor,
$X^4$: Chrom, Cobalt, Mangan, Zinn, Schwefel, Zink, Silicium, Selen, Eisen und/oder Nickel,
$X^5$: Kupfer, Silber, Rhodium und/oder Rhenium,
a: 0,3 bis 3
b: 0 bis 3
c: 0 bis 3
d: 0 bis 3
e: 0 bis 3
f: 0 bis 2 und
n: eine Zahl, die durch die Wertigkeit und Häufigkeit der von Sauerstoff verschiedenen Elementen in I bestimmt wird,
eingesetzt werden.

4. Verfahren nach Anspruch 1 oder 2, dadurch gekennzeichnet, daß als oxidische Katalysatoren Massen der allgemeinen Formel II

$$Mo_{12}P_aV_bX^1_cX^2_dX^3_eSb_fRe_gS_hO_n \qquad (II),$$

in der die Variablen folgende Bedeutung haben:

$X^1$: Kalium, Rubidium und/oder Cäsium,

$X^2$: Kupfer und/oder Silber,

$X^3$: Cer, Bor, Zirkonium, Mangan und/oder Wismut,

a: 0,5 bis 3,0

b: 0,01 bis 3,0

c: 0,2 bis 3,0

d: 0,01 bis 2,0

e: 0 bis 2,0

f: 0,01 bis 2,0

g: 0 bis 1,0

h: 0,001 bis 0,5 und

n: eine Zahl, die durch die Wertigkeit und Häufigkeit der von Sauerstoff verschiedenen Elemente in II bestimmt wird,

eingesetzt werden.

## Claims

1. A process for the catalytic gas-phase oxidation of methacrolein to methacrylic acid in a fixed-bed reactor with contacting tubes, at elevated temperature on catalytically active oxides with a conversion of methacrolein for a single pass of from 40 to 95 mol%, wherein the reaction temperature in the flow direction along the contacting tubes in a first reaction zone from entry of the starting reaction gases containing the reactants into the contacting tubes is from 280 to 340°C until a methacrolein conversion of from 20 to 40% is reached, and the reaction temperature is subsequently reduced by a total of from 5 to 45°C, abruptly or successively in steps or continuously along the contacting tubes until a methacrolein conversion of from 45 to 95% has been reached, with the proviso that the reaction temperature in this second reaction zone is not lower than 260°C.

2. A process as claimed in claim 1, wherein the reaction temperature of the reaction gases drops successively in the flow direction along the contacting tubes until they leave the contacting tubes.

3. A process as claimed in claim 1 or 2, wherein the oxidic catalysts are materials of the formula I

$$Mo_{12}P_aX_b^1 X_c^2 X_d^3 X_e^4 X_f^5 O_n \qquad (I)$$

where

$X^1$ is at least one alkali metal and/or alkaline earth metal,

$X^2$ is arsenic, tungsten, niobium and/or vanadium,

$X^3$ is antimony, bismuth, zirconium and/or boron,

$X^4$ is chromium, cobalt, manganese, tin, sulfur, zinc, silicon, selenium, iron and/or nickel,

$X^5$ is copper, silver, rhodium and/or rhenium,

a is from 0.3 to 3,

b is from 0 to 3,

c is from 0 to 3,

d is from 0 to 3,

e is from 0 to 3,

f is from 0 to 2 and

n is a number determined by the valency and frequency of the elements other than oxygen in the formula I.

4. A process as claimed in claim 1 or 2, wherein the oxidic catalysts are materials of the formula II

$$Mo_{12}P_aV_bX_c^1 X_d^2 X_e^3 Sb_fRe_gS_hO_n \qquad (II)$$

where

$X^1$ is potassium, rubidium and/or cesium,

$X^2$ is copper and/or silver,

6

X³   is cerium, boron, zirconium, manganese and/or bismuth,

a   is from 0.5 to 3.0,

b   is from 0.01 to 3.0,

c   is from 0.2 to 3.0,

d   is from 0.01 to 2.0,

e   is from 0 to 2.0,

f   is from 0.01 to 2.0,

g   is from 0 to 1.0,

h   is from 0.001 to 0.5 and

n   is a number determined by the valency and frequency of the elements other than oxygen in the formula II.

## Revendications

1. Procédé pour l'oxydation catalytique en phase gazeuse de la méthacroléine en acide méthacrylique dans un réacteur à lit fixe à tubes de contact, à température élevée, sur des oxydes catalytiquement actifs, avec une conversion de la méthacroléine de 40 à 95% molaires pour une passe unique, caractérisé en ce que la température réactionnelle dans la direction d'écoulement le long des tubes de contact dans une première zone réactionnelle depuis l'entrée des gaz réactionnels de départ contenant les réactifs dans les tubes de contact jusqu'à ce que soit atteinte une conversion de la méthacroléine de 20 à 40%, varie de 280 à 340°C et, ensuite, la température réactionnelle est abaissée brusquement ou, le long des tubes de contact, par échelons successifs ou en continu jusqu'à atteindre une conversion de la méthacroléine de 45 à 95°C, d'au total 5 à 45°C, avec la condition que la température réactionnelle dans cette seconde zone de réaction ne soit pas inférieure à 260°C.

2. Procédé suivant la revendication 1, caractérisé en ce que la température réactionnelle des gaz réactionnels dans la direction d'écoulement le long des tubes de contact diminue successivement jusqu'à leur sortie des tubes de contact.

3. Procédé suivant la revendication 1 ou 2, caractérisé en ce qu'à titre de catalyseurs oxydiques, on met en oeuvre des masses de la formule générale I

$$Mo_{12}P_aX^1_bX^2_cX^3_dX^4_eX^5_fO_n \quad (I),$$

dans laquelle

X¹:   représente au moins un métal alcalin et/ou métal alcalino-terreux,

X²:   représente l'arsenic, le tungstène, le niobium et/ou le vanadium,

X³:   représente l'antimoine, le bismuth, le zirconium et/ou le bore,

X⁴:   représente le chrome, le cobalt, le manganèse, l'étain, le soufre, le zinc, le silicium, le sélénium, le fer et/ou le nickel,

X⁵:   représente le cuivre, l'argent, le rhodium et/ou le rhénium,

a:   varie de 0,3 à 3

b:   varie de 0 à 3

c:   varie de 0 à 3

d:   varie de 0 à 3

e:   varie de 0 à 3

f:   varie de 0 à 2 et

n:   représente un nombre qui est déterminé par la valence et la fréquence des éléments différant de l'oxygène dans I.

4. Procédé suivant la revendication 1 ou 2, caractérisé en ce que, à titre de catalyseurs oxydiques, on met en oeuvre des masses de la formule générale II

$$Mo_{12}P_aV_bX^1_cX^2_dX^3_eSb_fRe_gS_hO_n \quad (II),$$

dans laquelle

X¹:   représente le potassium, le rubidium et/ou le césium,

X²:   représente le cuivre et/ou l'argent,

X³: représente le cérium, le bore, le zirconium, le manganèse et/ou le bismuth,

a: varie de 0,5 à 3,0

b: varie de 0,01 à 3,0

c: varie de 0,2 à 3,0

d: varie de 0,01 à 2,0

e: varie de 0 à 2,0

f: varie de 0,01 à 2,0

g: varie de 0 à 1,0

h: varie de 0,001 à 0,5 et

n: représente un nombre déterminé par la valence et la fréquence d'éléments différant de l'oxygène dans II.